# EUROPÄISCHE PATENTSCHRIFT

(11) **EP 2 697 376 B1**
(45) Veröffentlichungstag und Bekanntmachung des Hinweises auf die Patenterteilung: **31.05.2017**
(21) Anmeldenummer: 12713714.9
(22) Anmeldetag: 05.04.2012
(51) Int. Cl.: C12N 15/67, C12N 9/48, C12P 21/02

(54) **EXPRESSIONSVERFAHREN MIT EINER HILFSPROTEASE**
EXPRESSION METHOD WITH A HELPER PROTEASE
PROCÉDÉ D'EXPRESSION AVEC UNE PROTÉASE AUXILIAIRE

(30) Priorität: 13.04.2011 DE 102011007313
(43) Veröffentlichungstag der Anmeldung: 19.02.2014
(73) Patentinhaber: BASF SE, 67056 Ludwigshafen am Rhein (DE)
(72) Erfinder: MAKSYM, Lukas, 50674 Köln (DE); KNAB, Ramona, 41542 Dormagen (DE); EVERS, Stefan, 40822 Mettmann (DE); MAURER, Karl-Heinz, 40699 Erkrath (DE); BONGAERTS, Johannes, 41541 Dormagen (DE)
(74) Vertreter: BASF IP Association
(86) Internationale Anmeldenummer: PCT/EP2012/056262
(87) Internationale Veröffentlichungsnummer: WO 2012/139964

(56) Entgegenhaltungen:
- EP-A1- 1 873 251
- EP-A2- 1 921 148
- WO-A1-89/06279
- WO-A1-91/02792
- WO-A1-2004/085649
- WO-A1-2009/094084
- WO-A2-03/062381
- US-A1- 2008 199 443
- GODDETTE D W ET AL: "The crystal structure of the Bacillus lentus alkaline protease, subtilisin BL, at 1.4 A resolution", JOURNAL OF MOLECULAR BIOLOGY, ACADEMIC PRESS, UNITED KINGDOM, Bd. 228, Nr. 2, 20. November 1992 (1992-11-20), Seiten 580-595, XP024015190, ISSN: 0022-2836, DOI: 10.1016/0022-2836(92)90843-9 [gefunden am 1992-11-20]
- FUJIO KAWAMURA ET AL: "Construction of a Bacillus subtilis Double Mutant Deficient in Extracellular Alkaline and Neutral Proteases", JOURNAL OF BACTERIOLOGY, AMERICAN SOCIETY FOR MICROBIOLOGY, US, vol. 160, no. 1, 1 October 1984 (1984-10-01), pages 442-444, XP001314015, ISSN: 0021-9193
- WU X-C ET AL: "ENGINEERING A BACILLUS SUBTILIS EXPRESSION-SECRETION SYSTEM WITH A STRAIN DEFICIENT IN SIX EXTRACELLULAR PROTEASES", JOURNAL OF BACTERIOLOGY, AMERICAN SOCIETY FOR MICROBIOLOGY, US, vol. 173, no. 16, 1 August 1991 (1991-08-01), pages 4952-4958, XP009005082, ISSN: 0021-9193
- Anonymous: "Kanamycin - Wikipedia, the free encyclopedia", , 2 November 2015 (2015-11-02), XP055230287, Retrieved from the Internet: URL:https://en.wikipedia.org/w/index.php?t itle=Kanamycin&printable=yes [retrieved on 2015-11-23]

### SEQUENCE LISTING

<110> Henkel AG & Co. KGaA
<120> Expressionsverfahren
<130> H 08980 PCT
<150> DE102011007313.2
   <151> 2011-04-13
<160> 12
<170> PatentIn version 3.3
<210> 1
   <211> 269
   <212> PRT
   <213> Bacillus sp.
<400> 1
   Ala Gln Thr Ile Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Met Ala Pro Gly Val Asn Ile Gln Ser Thr Tyr Pro Gly Ser Thr Tyr
           195                 200                 205
   Ala Ser Asp Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 2
   <211> 274
   <212> PRT
   <213> Bacillus sp.
<400> 2
   Ala Gln Thr Val Pro Tyr Gly Ile Pro Leu Ile Lys Ala Asp Lys Val
   1               5                   10                  15
   Gln Ala Gln Gly Phe Lys Gly Ala Asn Val Lys Val Ala Val Leu Asp
               20                  25                  30
   Thr Gly Ile Gln Ala Ser His Pro Asp Leu Asn Val Val Gly Gly Ala
           35                  40                  45
   Ser Phe Val Ala Gly Glu Ala Tyr Asn Thr Asp Gly Asn Gly His Gly
       50                  55                  60
   Thr His Val Ala Gly Thr Val Ala Ala Leu Asp Asn Thr Thr Gly Val
   65                  70                  75                  80
   Leu Gly Val Ala Pro Ser Val Ser Leu Tyr Ala Val Lys Val Leu Asn
                   85                  90                  95
   Ser Ser Gly Ser Gly Ser Tyr Ser Gly Ile Val Ser Gly Ile Glu Trp
               100                 105                 110
   Ala Thr Thr Asn Gly Met Asp Val Ile Asn Met Ser Leu Gly Gly Ala
           115                 120                 125
   Ser Gly Ser Thr Ala Met Lys Gln Ala Val Asp Asn Ala Tyr Ala Arg
           130                 135                 140
   Gly Val Val Val Val Ala Ala Ala Gly Asn Ser Gly Ser Ser Gly Asn
   145                 150                 155                 160
   Thr Asn Thr Ile Gly Tyr Pro Ala Lys Tyr Asp Ser Val Ile Ala Val
                   165                 170                 175
   Gly Ala Val Asp Ser Asn Ser Asn Arg Ala Ser Phe Ser Ser Val Gly
               180                 185                 190
   Ala Glu Leu Glu Val Met Ala Pro Gly Ala Gly Val Tyr Ser Thr Tyr
           195                 200                 205
   Pro Thr Asn Thr Tyr Ala Thr Leu Asn Gly Thr Ser Met Ala Ser Pro
       210                 215                 220
   His Val Ala Gly Ala Ala Ala Leu Ile Leu Ser Lys His Pro Asn Leu
   225                 230                 235                 240
   Ser Ala Ser Gln Val Arg Asn Arg Leu Ser Ser Thr Ala Thr Tyr Leu
                   245                 250                 255
   Gly Ser Ser Phe Tyr Tyr Gly Lys Gly Leu Ile Asn Val Glu Ala Ala
               260                 265                 270
   Ala Gln
<210> 3
   <211> 29
   <212> DNA
   <213> Artificial
<220>
   <223> Primer aprE1
<400> 3
   aaactgcagc gtaccggctc cttgaaagg 29
<210> 4
   <211> 50
   <212> DNA
   <213> Artificial
<220>
   <223> Primer aprE2
<400> 4
   gagaagacta agcttcccga ggatcccgaa tgacaggaga ttgctccatc 50
<210> 5
   <211> 63
   <212> DNA
   <213> Artificial
<220>
   <223> Primer aprE3
<400> 5
   ttcgggatcc tcgggaagct tagtcttctc attctgatga aggttgttca atattttgaa 60
   tcc 63
<210> 6
   <211> 30
   <212> DNA
   <213> Artificial
<220>
   <223> Primer aprE4
<400> 6
   aaatctagat agacctcgaa gaggagaagc 30
<210> 7
   <211> 32
   <212> DNA
   <213> Artificial
<220>
   <223> Primer tet5
<400> 7
   gcggatccaa acgggccata ttgttgtata ag 32
<210> 8
   <211> 35
   <212> DNA
   <213> Artificial
<220>
   <223> Primer tet6
<400> 8
   ccaagcttct ctcgtatctt ttattcagca atcgc 35
<210> 9
   <211> 50
   <212> DNA
   <213> Artificial
<220>
   <223> Primer aprE7
<400> 9
   cgaaagtatg aatagaccgc ttcagcctgg cagggaaaga ggtcccgagg 50
<210> 10
   <211> 50
   <212> DNA
   <213> Artificial
<220>
   <223> Primer aprE8
<400> 10
   ctgccgctca ataacatatt ctaacaaata gcatatagaa aaagctagtg 50
<210> 11
   <211> 50
   <212> DNA
   <213> Artificial
<220>
   <223> Primer ziel9
<400> 11
   ctgccaggct gaagcggtct attcatactt tcgaactgaa catttttcta 50
<210> 12
   <211> 50
   <212> DNA
   <213> Artificial
<220>
   <223> Primer ziel10
<400> 12
   atttgttaga atatgttatt gatcggcagc ttcgacattg atcagacctt 50

## Beschreibung

Die Erfindung liegt auf dem Gebiet der Biotechnologie, insbesondere der mikrobiellen Proteinsynthese. Die Erfindung betrifft insbesondere ein Verfahren zur Herstellung von Proteinen durch genetisch modifizierte Mikroorganismen und schlägt ferner Mikroorganismen vor, die in derartigen Verfahren Verwendung finden. Die Erfindung betrifft ferner Verwendungen derartiger Mikroorganismen zur Proteinherstellung.

Für die Herstellung von Wertstoffen können Mikroorganismen eingesetzt werden.

Wertstoffe sind beispielsweise niedermolekulare Verbindungen, etwa Nahrungsmittelergänzungsstoffe oder pharmazeutisch wirksame Verbindungen, oder Proteine, für welche aufgrund ihrer Diversität wiederum ein großes technisches Einsatzgebiet besteht. Im ersten Fall werden die Stoffwechseleigenschaften der betreffenden Mikroorganismen zur Herstellung der Wertstoffe ausgenutzt und/oder verändert; im zweiten Fall werden vorzugsweise Mikroorganismen eingesetzt, die die Gene der interessierenden Proteine exprimieren.

Für die großtechnische, biotechnologische Produktion werden die betreffenden Mikroorganismen in Fermentern kultiviert, die den Stoffwechseleigenschaften der Mikroorganismen entsprechend ausgestaltet sind. Während der Kultivierung verstoffwechseln die Mikroorganismen das angebotene Substrat und bilden das gewünschte Produkt, das nach Beendigung der Fermentation üblicherweise von den Produktionsorganismen abgetrennt wird und aus dem Fermenterbrei und/oder dem Fermentationsmedium aufgereinigt und/oder aufkonzentriert wird. Bei der fermentativen Produktion von Proteinen werden typischerweise komplexe proteinreiche Rohstoffe als Substrat neben einer Kohlenstoffquelle (typischerweise Glukose) eingesetzt. Die Proteinproduktion entspricht damit einer Biotransformation von Substratprotein zum Zielprotein. Dies erfordert die vollständige Hydrolyse des Substratproteins in die einzelnen Aminosäuren, die dann zur Biosynthese des Zielproteins zur Verfügung stehen.

Zur Fermentation von Mikroorganismen besteht folglich ein reichhaltiger Stand der Technik, der von der Optimierung der betreffenden Stämme, beispielsweise hinsichtlich der Bildungsrate und der Nährstoffausnutzung, über die technische Gestaltung der Fermenter bis hin zur Gewinnung der Wertstoffe aus den betreffenden Mikroorganismen und/oder dem Fermentationsmedium reicht.

Üblicherweise sind möglichst hohe Produktausbeuten bei der mikrobiellen Fermentation wünschenswert. Beispielsweise offenbart die internationale Patentanmeldung WO 91/02792 die verbesserte fermentative Produktion einer alkalischen Protease aus *Bacillus lentus* in einem optimierten *Bacillus licheniformis* Stamm unter der Kontrolle genregulatorischer Sequenzen aus *Bacillus licheniformis,* insbesondere des *Bacillus licheniformis*-Promotors.

In den Veröffentlichungen von Wu et al. (J. Bacteriol. 173(16), 4952-8 (1991); Appl. Environ. Microbiol. 68(7), 3261-9 (2002)) wird vorgeschlagen, von dem Mikroorganismus endogen exprimierte Proteasen auszuschalten, um die Ausbeute an Zielprotein zu erhöhen. Hierdurch soll ein proteolytischer Abbau des Zielproteins verhindert und das Synthesevermögen der Mikroorganismen auf das gewünschte Produkt fokussiert werden.

WO 2009/094084 beschreibt einen *Bacillus* Mikroorganismus, der eine mutierte Protease und das Protein YmaH exprimiert.

EP 1921148 beschreibt einen Mikroorganismus, der eine mutierte Protease exprimiert, wobei die entsprechenden Klone mithilfe eines Resistenzgens selektiert wurden.

Es besteht weiterhin ein hoher Bedarf an mikrobiellen Fermentationsverfahren, die eine hohe Produktausbeute ermöglichen. Überraschenderweise und im Gegensatz zu den vorstehend genannten Veröffentlichungen wurde nun gefunden, dass gerade die zusätzliche Expression einer bestimmten Hilfsprotease für die Produktausbeute vorteilhaft ist.

Der vorliegenden Erfindung liegt die Aufgabe zugrunde, die Produktausbeute, insbesondere eines Proteins, in einer mikrobiellen Fermentation zu erhöhen.

Gegenstand der Erfindung ist ein Verfahren zur Herstellung eines Proteins durch einen Mikroorganismus umfassend die Verfahrensschritte
(a) Einbringen eines ersten Expressionskonstruktes in einen Mikroorganismus, welches für das Protein codiert;
(b) Einbringen eines zweiten Expressionskonstruktes in den Mikroorganismus, welches für eine Hilfsprotease codiert, die sich von dem Protein unterscheidet und die eine Aminosäuresequenz umfasst, die zu der in SEQ ID NO. 1 angegebenen Aminosäuresequenz zu mindestens 65% identisch ist, wobei die Hilfsprotease eine proteolytische Aktivität aufweist und mindestens eine chromosomal codierte Protease in dem Mikroorganismus ersetzt;
(c) Exprimieren des Proteins und der Hilfsprotease in dem Mikroorganismus, wobei das Protein eine Protease, Amylase, Cellulase, Hemicellulase, Mannanase, Tannase, Xylanase, Xanthanase, Xylaoglucanase, [beta]-Glucosidase, Pektinase, Carragenase, Perhydrolase, Oxidase, Oxidoreduktase oder eine Lipase ist.
   Ein erfindungsgemäßes Verfahren umfasst optional ferner den weiteren Verfahrensschritt
(d) Kultivieren des Mikroorganismus.

In bevorzugten erfindungsgemäßen Ausgestaltungen handelt es sich bei einem erfindungsgemäßen Verfahren folglich um ein Fermentationsverfahren.

Das Einbringen und Exprimieren der Hilfsprotease in den Mikroorganismus führt unerwarteterweise nicht dazu, dass die Produktausbeute an Protein (Zielprotein) abnimmt, beispielsweise auf Grund der proteolytischen Aktivität der exprimierten Hilfsprotease, die synthetisiertes Zielprotein abbauen könnte. Vielmehr bewirkt die Expression der Hilfsprotease, dass die Produktausbeute an Protein gesteigert wird. In bevorzugten Ausführungsformen der Erfindung ist die Hilfsprotease an der Hydrolyse von Substratprotein beteiligt und bewirkt, vorzugsweise zusammen mit weiteren, chromosomal codierten Proteasen (= Hintergrundproteasen) des Mikroorganismus, einen verbesserten Aufschluss des Substratproteins, so dass mehr der nötigen Vorläufermoleküle, insbesondere Aminosäuren, pro Zeiteinheit für die Synthese des Zielproteins im Produktionsprozess zur Verfügung stehen. In derartigen erfindungsgemäßen Ausgestaltungen ergänzen sich die Substratspezifität der Hintergrundproteasen und der Hilfsprotease demnach vorteilhaft hinsichtlich des Substrataufschlusses durch den Mikroorganismus.

Diesbezüglich ist es in weiteren bevorzugten Ausführungsformen der Erfindung nicht notwendig, dass die Hilfsprotease einen signifikanten Anteil am Fermentationsprodukt besitzt. Die Hilfsprotease wirkt diesbezüglich förderlich auf die Expression des Proteins und folglich auf die Produktausbeute an Protein, ihr Anteil im Fermentationsprodukt ist aber vorzugsweise klein oder gar nicht erfassbar. Vorzugsweise beträgt ihr Anteil im Fermentationsprodukt weniger als 25% und zunehmend bevorzugt weniger als 20 Gew.-%, 15 Gew.-%, 10 Gew.-%, 8 Gew.-%, 7 Gew.-%, 6 Gew.-%, 5 Gew.-%, 2,5 Gew.-%, 2 Gew.-%, 1,5 Gew.-%, 1 Gew.-%, 0,5 Gew.-%. 0,1 Gew.-% und 0,05 Gew.-%. Das Fermentationsprodukt ist diesbezüglich diejenige Zusammensetzung, in welcher das Protein enthalten ist, nachdem ein erfindungsgemäßes Verfahren durchgeführt wurde, der Mikroorganismus in einem Kulturmedium kultiviert wurde und optional der Mikroorganismus aufgeschlossen wurde, um das Protein aus diesem freizusetzen, sofern es nicht von diesem sezerniert wurde. Vorzugsweise wurden von dem Fermentationsprodukt die Mikroorganismen bzw. ihre Bruchstücke abgetrennt.

In einer bevorzugten Ausgestaltung handelt es sich bei dem erfindungsgemäßen Verfahren folglich um ein Verfahren zur Steigerung der Expression eines Proteins in einem Mikroorganismus. Eine gesteigerte Expression des Proteins liegt vor, wenn durch ein erfindungsgemäßes Verfahren eine größere Menge an Protein erhalten wird im Vergleich mit einem gleichartigen Verfahren, das sich von einem erfindungsgemäßen Verfahren lediglich durch den Verzicht auf den Verfahrensschritt b) unterscheidet, wodurch in Verfahrensschritt c) folglich keine Hilfsprotease in dem Mikroorganismus exprimiert wird. Beide zu vergleichenden Verfahren werden diesbezüglich unter gleichen Bedingungen, für die gleiche Dauer und mit Mikroorganismen durchgeführt, die sich lediglich durch die An- bzw. Abwesenheit der Hilfsprotease unterscheiden.

Ein Expressionskonstrukt ist eine Nukleinsäuresequenz, die bewirkt, dass das Protein bzw. die Hilfsprotease in dem Mikroorganismus exprimiert werden kann. Es umfasst die genetische Information, also diejenige Nukleinsäuresequenz (Gen), die für das Protein bzw. für die Hilfsprotease codiert. Die Expression einer Nukleinsäuresequenz ist dessen Übersetzung in das bzw. die von dieser Sequenz codierte(n) Genprodukt(e), also in ein Polypeptid (Protein) bzw. in mehrere Polypeptide (Proteine). Die Begriffe Polypeptid und Protein werden in der vorliegenden Anmeldung synonym verwendet. Im Sinne der vorliegenden Erfindung bezeichnet Expression folglich die Biosynthese von Ribonucleinsäure (RNA) und Proteinen aus den genetischen Informationen. In der Regel umfasst die Expression die Transkription, also die Synthese einer Boten ("messenger")-Ribonukleinsäure (mRNA) anhand der DNA (Desoxyribonukleinsäure)- Sequenz des Gens und deren Translation in die entsprechende Polypeptidkette, die gegebenenfalls noch posttranslational modifiziert werden kann. Das Exprimieren eines Proteins beschreibt folglich die Biosynthese desselben aus den genetischen Informationen, die erfindungsgemäß in dem Mikroorganismus vorliegen.

Ein Expressionskonstrukt umfasst ferner mindestens eine Nukleinsäuresequenz, vorzugsweise DNA, mit einer Steuerungsfunktion für die Expression der für das Protein bzw. die Hilfsprotease codierenden Nukleinsäuresequenz (sog. genregulatorische Sequenz). Eine genregulatorische Sequenz ist hierbei jede Nukleinsäuresequenz, deren Anwesenheit in dem jeweiligen Mikroorganismus die Transkriptionshäufigkeit derjenigen Nukleinsäuresequenz beeinflusst, vorzugsweise erhöht, die für das Protein bzw. die Hilfsprotease codiert. Vorzugsweise handelt es sich um eine Promotor-Sequenz, da eine derartige Sequenz für die Expression einer Nukleinsäuresequenz wesentlich ist. Ein Expressionskonstrukt kann aber auch noch weitere genregulatorische Sequenzen umfassen, beispielsweise eine oder mehrere Enhancer-Sequenzen. Ein Expressionskonstrukt im Rahmen der Erfindung umfasst folglich mindestens eine funktionelle Einheit aus Gen und Promotor. Sie kann, muss jedoch nicht notwendigerweise, als physische Einheit vorliegen.

Das Vorhandensein von mindestens einem Promotor ist für ein Expressionskonstrukt wesentlich. Unter einem Promotor wird demnach eine DNA-Sequenz verstanden, die die regulierte Expression eines Gens ermöglicht. Natürlicherweise ist eine Promotorsequenz ein Bestandteil eines Gens und liegt oftmals an dessen 5'-Ende und somit vor dem RNA-kodierenden Bereich.

Vorzugsweise liegt die Promotorsequenz in einem Expressionskonstrukt 5'-wärts von der für das Protein bzw. die Hilfsprotease codierenden Nukleinsäuresequenz. Die wichtigste Eigenschaft eines Promotors ist die spezifische Wechselwirkung mit mindestens einem DNA-bindenden Protein bzw. Polypeptid, welches den Start der Transkription des Gens durch eine RNA-Polymerase vermittelt und als Transkriptionsfaktor bezeichnet wird. Häufig sind mehrere Transkriptionsfaktoren und/oder weitere Proteine am Start der Transkription durch eine RNA-Polymerase beteiligt. Ein Promotor ist demnach vorzugsweise eine DNA-Sequenz mit Promotoraktivität, d.h. eine DNA-Sequenz, an die mindestens ein Transkriptionsfaktor zur Initiation der Transkription eines Gens zumindest transient bindet. Die Stärke eines Promotors ist messbar über die Transkriptionshäufigkeit des exprimierten Gens, also über die Anzahl der pro Zeiteinheit erzeugten RNA-Moleküle, insbesondere mRNA-Moleküle. Ein Promotor eines Expressionskonstruktes kann ein eigener Promotor des Mikroorganismus sein. Eine derartige Promotorsequenz ist folglich natürlicherweise in dem Mikroorganismus vorhanden. Alternativ kann ein Promotor eines Expressionskonstruktes auch rekombinant in den Mikroorganismus eingebracht worden sein. Entsprechendes gilt auch für alle weiteren genregulatorischen Sequenzen, die ein Expressionskonstrukt aufweisen kann.

Das erste Expressionskonstrukt codiert für ein Protein. Es umfasst folglich eine Nukleinsäuresequenz, die für dieses Protein codiert. Hierfür ist grundsätzlich eine beliebige Nukleinsäuresequenz verwendbar, die in ein Protein translatiert werden kann. Hierbei handelt es sich um dasjenige Protein, das mit Hilfe eines erfindungsgemäßen Verfahrens hergestellt werden soll (Zielprotein). Erfindungsgemäß handelt es sich um ein Enzym wie nachstehend beschrieben. Das zweite Expressionskonstrukt codiert für die Hilfsprotease. Die Hilfsprotease unterscheidet sich von dem Protein, d.h. sie und das Protein weisen unterschiedliche Aminosäuresequenzen auf. Die Hilfsprotease umfasst eine Aminosäuresequenz, die zu der in SEQ ID NO. 1 angegebenen Aminosäuresequenz zu mindestens 65% und zunehmend bevorzugt zu mindestens 70%, 72%, 74%, 76%, 78%, 80%, 81%, 82%, 83%, 84%, 85%, 86%, 87%, 88%, 89%, 90%, 91%, 92%, 93%, 94%, 95%, 96%, 97%, 98%, 99% und ganz besonders bevorzugt zu 100% identisch ist. Besonders bevorzugt weist die Hilfsprotease eine Aminosäuresequenz auf, die zu der in SEQ ID NO. 1 angegebenen Aminosäuresequenz zu mindestens 65% identisch und zunehmend bevorzugt zu mindestens 70%, 72%, 74%, 76%, 78%, 80%, 81%, 82%, 83%, 84%, 85%, 86%, 87%, 88%, 89%, 90%, 91%, 92%, 93%, 94%, 95%, 96%, 97%, 98%, 99% und ganz besonders bevorzugt zu 100% identisch ist.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Im Rahmen der vorliegenden Erfindung werden Sequenzvergleiche und Alignments bevorzugt mit dem Computer-Programm Vector NTI® Suite 10.3 (Invitrogen Corporation, 1600 Faraday Avenue, Carlsbad, Kalifornien, USA) mit den vorgegebenen Standard (Default)-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen, angegeben. Der weiter gefasste Begriff der Homologie bezieht bei Aminosäuresequenzen konservierte Aminosäure-Austausche in die Betrachtung mit ein, also Aminosäuren mit ähnlichen Eigenschaften, da diese innerhalb des Proteins meist ähnliche Aktivitäten bzw. Funktionen ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Sie weisen oftmals gleiche oder ähnliche Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz. Bei Proteinen, insbesondere bei Enzymen und hierunter besonders bei Proteasen, beziehen sich die Angaben ferner auf das jeweils reife (mature) Protein, soweit nicht anders angegeben. Ohne anders lautende Angaben ist die Sequenzbetrachtung folglich immer auf das reife, fertig prozessierte Protein gerichtet, selbst wenn von dem zugehörigen Gen eine immature Form codiert wird, die nach der Translation noch zur reifen Form prozessiert wird.

Die Hilfsprotease weist ferner eine proteolytische Aktivität auf. Sie ist folglich katalytisch aktiv, d.h. es handelt sich um aktives Enzym. Die Bestimmung der Enzymaktivität kann diesbezüglich - abgestimmt auf den jeweiligen Enzymtyp - in fachüblicher Art und Weise erfolgen. Methoden zur Aktivitätsbestimmung sind dem Fachmann auf dem Gebiet der Enzymtechnologie geläufig und werden von ihm routinemäßig angewendet. Verfahren zur Bestimmung der Proteaseaktivität sind beispielsweise offenbart in Tenside, Band 7 (1970), S. 125-132. Die proteolytische Aktivität kann ferner bestimmt werden über die Freisetzung des Chromophors para-Nitroanilin (pNA) aus dem Substrat suc-L-Ala-L-Ala-L-Pro-L-Phe-p-Nitroanilid (suc-AAPF-pNA). Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6 und einer Wellenlänge von 410 nm. Die Messzeit beträgt 5 min. bei einem Messintervall von 20s bis 60s. Die Proteaseaktivität wird vorzugsweise in PE (Protease-Einheiten) angegeben.

Nukleinsäuren und Expressionskonstrukte können über an sich bekannte Verfahren zur Veränderung von Nukleinsäuren erzeugt werden. Solche sind beispielsweise in einschlägigen Handbüchern wie dem von Fritsch, Sambrook und Maniatis, "Molecular cloning: a laboratory manual", Cold Spring Harbour Laboratory Press, New York, 1989, dargestellt und dem Fachmann auf dem Gebiet der Biotechnologie geläufig. Beispiele für solche Verfahren sind die chemische Synthese oder die Polymerase-Kettenreaktion (PCR), optional in Verbindung mit weiteren molekularbiologischen und/oder chemischen bzw. biochemischen Standardmethoden.

Die vorliegende Erfindung eignet sich insbesondere für die rekombinante Herstellung von Proteinen, insbesondere Enzymen. Hierfür werden das erste und das zweite Expressionskonstrukt in einen Mikroorganismus eingebracht, vorzugsweise durch Transformation. Diesbezüglich erfolgt die Einschleusung des jeweiligen Expressionskonstruktes oder Teilen hiervon vorzugsweise über Vektoren, insbesondere Expressionsvektoren. Es ist aber auch möglich, dass das nur Teile des Expressionskonstruktes, vorzugsweise zumindest die Nukleinsäure, die für das Protein oder die Hilfsprotease codiert, in den Mikroorganismus derart eingebracht werden, dass das fertige Expressionskonstrukt erst in dem Mikroorganismus entsteht. Dies kann beispielsweise durch einen Vektor erfolgen, der bewirkt, dass das Gen für das Protein und/oder das Gen für die Hilfsprotease in der Wirtszelle in ein bereits vorhandenes genetisches Element wie das Chromosom, die chromosomale DNA oder andere Vektoren eingefügt werden kann, so dass beispielsweise ein endogener Promotor für die Expression des Gens für das Protein oder des Gens für die Hilfsprotease genutzt wird. Insbesondere kann das Gen, das für die Hilfsprotease codiert, auf diese Weise mit einem endogenen Promotor des Mikroorganismus funktionell verknüpft werden und stellt dann das zweite Expressionskonstrukt dar. Der Begriff des Einbringens umfasst folglich die Möglichkeit, dass ein Expressionskonstrukt vollständig in den Mikroorganismus eingebracht, vorzugsweise transformiert, wird, aber auch die Möglichkeit, dass nur ein Teil des Expressionskonstruktes, besonders bevorzugt die Nukleinsäure, die für die Hilfsprotease codiert, in den Mikroorganismus eingebracht, vorzugsweise transformiert, wird und das vollständige Expressionskonstrukt erst in dem Mikroorganismus entsteht. Zumindest ein Teil des Expressionskonstruktes wird im Rahmen der Erfindung aber immer in den Mikroorganismus eingebracht.

Vektoren sind dem Fachmann auf dem Gebiet der Biotechnologie bekannt. Sie sind insbesondere bei der Verwendung in Bakterien spezielle Plasmide, also zirkulare genetische Elemente. Im Rahmen der vorliegenden Erfindung sind die Expressionskonstrukte vorzugsweise in einen Vektor kloniert. Zu den Vektoren können beispielsweise solche gehören, die sich von bakteriellen Plasmiden, von Viren oder von Bacteriophagen ableiten, oder überwiegend synthetische Vektoren oder Plasmide mit Elementen verschiedenster Herkunft. Mit den weiteren jeweils vorhandenen genetischen Elementen vermögen Vektoren sich in den Mikroorganismen über mehrere Generationen hinweg als stabile Einheiten zu etablieren. Es ist dabei im Sinne der Erfindung unerheblich, ob sie sich extrachomosomal als eigene Einheiten etablieren oder in ein Chromosom bzw. chromosomale DNA integrieren. Welches der zahlreichen Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Mikroorganismen sein.

Expressionsvektoren können ferner durch Änderungen der Kulturbedingungen wie beispielsweise die Zelldichte oder die Zugabe von bestimmten Verbindungen regulierbar sein. Ein Beispiel für eine solche Verbindung ist das Galactose-Derivat Isopropyl-ß-D-thiogalactopyranosid (IPTG), welches als Aktivator des bakteriellen Lactose-Operons (lac-Operons) verwendet wird.

In einer weiteren Ausführungsform der Erfindung ist ein erfindungsgemäßes Verfahren dadurch gekennzeichnet, dass das Protein nicht natürlicherweise in dem Mikroorganismus vorhanden ist und/oder die Hilfsprotease nicht natürlicherweise in dem Mikroorganismus vorhanden ist.

Nicht natürlicherweise vorhanden bedeutet in diesem Zusammenhang, dass das Protein bzw. die Hilfsprotease kein eigenes Protein bzw. Enzym des Mikroorganismus ist. Das Protein bzw. die Hilfsprotease kann folglich in dem Mikroorganismus nicht von einer Nukleinsäuresequenz exprimiert werden, die Teil der chromosomalen DNA des Mikroorganismus in seiner Wildtyp-Form ist. Das Protein bzw. die Hilfsprotease und/oder die hierfür jeweils codierende Nukleinsäuresequenz ist folglich in der Wildtyp-Form des Mikroorganismus nicht vorhanden und/oder kann aus der Wildtyp-Form des Mikroorganismus nicht aus diesem isoliert werden. Vorzugsweise ist ein nicht natürlicherweise in dem Mikroorganismus vorhandenes Protein bzw. eine nicht natürlicherweise in dem Mikroorganismus vorhandene Hilfsprotease bzw. die jeweilige hierfür codierende Nukleinsäuresequenz mit Hilfe gentechnischer Verfahren in den Mikroorganismus gezielt eingebracht worden, so dass der Mikroorganismus um das Protein bzw. die Hilfsprotease bzw. die jeweilige hierfür codierende Nukleinsäuresequenz bereichert worden ist. Jedoch kann ein Protein bzw. die Hilfsprotease durchaus natürlicherweise in einem anderen Mikroorganismus vorhanden sein - relevant für die Betrachtung ist ausschließlich der in dem Verfahren eingesetzte Mikroorganismus.

Es können sowohl das Protein als auch die Hilfsprotease nicht natürlicherweise in dem Mikroorganismus vorhanden sein. In einer alternativen Ausgestaltung kann aber das Protein natürlicherweise in dem Mikroorganismus vorhanden und die Hilfsprotease nicht natürlicherweise in dem Mikroorganismus vorhanden sein. In einer weiteren alternativen Ausgestaltung kann auch das Protein nicht natürlicherweise in dem Mikroorganismus vorhanden und die Hilfsprotease natürlicherweise in dem Mikroorganismus vorhanden sein.

Das erfindungsgemäße Verfahren ist dadurch gekennzeichnet, dass die Hilfsprotease mindestens eine chromosomal codierte Protease in dem Mikroorganismus ersetzt. Hierbei wird mindestens eine vorliegende chromosomale Protease durch eine heterologe Protease, die als Hilfsprotease fungiert, ausgetauscht. Vorzugsweise wird die Nukleinsäuresequenz, die für die Hilfsprotease codiert, in eine chromosomale Nukleinsäuresequenz eingefügt, die für eine Protease des Mikroorganismus codiert. Hierdurch wird die chromosomal codierte Protease funktionell inaktiviert. Anstelle der chromosomal codierten Protease exprimiert der Mikroorganismus die Hilfsprotease. Für die Expression der Hilfsprotease kann der ursprüngliche, für die chromosomal codierte Protease genutzte Promotor verwendet werden, so dass das zweite Expressionskonstrukt in diesem Fall neben der für die Hilfsprotease codierenden Nukleinsäuresequenz die Promotorsequenz der chromosomal codierten Protease, d.h. eine natürliche Promotorsequenz des Mikroorganismus, umfasst. Alternativ kann ein für die Hilfsprotease eigens vorgesehener Promotor verwendet werden, so dass das zweite Expressionskonstrukt in diesem Fall neben der für die Hilfsprotease codierenden Nukleinsäuresequenz nicht die Promotorsequenz der chromosomal codierten Protease umfasst. Vorzugsweise umfasst das zweite Expressionskonstrukt in diesem Fall eine nicht natürliche Promotorsequenz des Mikroorganismus. Sofern ein für die Hilfsprotease eigens vorgesehener Promotor verwendet wird, wurde dieser vorzugsweise zusammen mit der Nukleinsäuresequenz, die für die Hilfsprotease codiert, in die chromosomale Nukleinsäuresequenz eingefügt, die für eine Protease des Mikroorganismus codiert. In einer weiteren alternativen Ausgestaltung können für die Expression der Hilfsprotease sowohl der für die chromosomal codierte Protease genutzte Promotor als auch ein für die Hilfsprotease eigens vorgesehener Promotor verwendet werden.

In einer weiteren Ausführungsform der Erfindung ist ein erfindungsgemäßes Verfahren dadurch gekennzeichnet, dass die Hilfsprotease in dem Mikroorganismus zusätzlich zu den chromosomal codierten Proteasen exprimiert wird. Hierbei wird in dem Mikroorganismus eine zusätzliche proteolytische Aktivität bereitgestellt durch eine heterologe Protease, die als erfindungsgemäße Hilfsprotease fungiert. Das zweite Expressionskonstrukt umfasst folglich vorzugsweise mindestens die Nukleinsäuresequenz, die für die Hilfsprotease codiert, sowie einen für die Expression der Hilfsprotease vorgesehenen Promotor. Vorzugsweise umfasst das zweite Expressionskonstrukt in diesem Fall eine nicht natürliche Promotorsequenz des Mikroorganismus.

In einer besonders bevorzugten Ausführungsform ist ein erfindungsgemäßes Verfahren dadurch gekennzeichnet, dass die Hilfsprotease mindestens eine chromosomal codierte Protease in dem Mikroorganismus ersetzt, die eine Aminosäuresequenz umfasst, die zu der in SEQ ID NO. 2 angegebenen Aminosäuresequenz zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% und ganz besonders bevorzugt zu 100% identisch ist. Besonders bevorzugt weist die chromosomal codierte Protease eine Aminosäuresequenz auf, die zu der in SEQ ID NO. 2 angegebenen Aminosäuresequenz zu mindestens 80% identisch und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% und ganz besonders bevorzugt zu 100% identisch ist. Besonders bevorzugt wird eine derartige Protease in einem Mikroorganismus ersetzt, der ein Bakterium ist, insbesondere eines der Gattung *Bacillus* und hierunter vorzugsweise in *Bacillus licheniformis.*

In einer weiteren Ausführungsform der Erfindung ist ein erfindungsgemäßes Verfahren dadurch gekennzeichnet, dass die Expression des Proteins durch die Expression der Hilfsprotease verstärkt wird. Wie vorstehend bereits erläutert bewirkt die Expression der Hilfsprotease in bevorzugten erfindungsgemäßen Ausgestaltungen, dass die Produktausbeute an Protein gesteigert wird. Dies wird durch eine gesteigerte Expression, d.h. eine gesteigerte Synthese des Proteins durch den Mikroorganismus, erreicht. Die Hilfsprotease ist an der Hydrolyse von Substratprotein beteiligt und bewirkt, gegebenenfalls zusammen mit weiteren, chromosomal codierten Proteasen des Mikroorganismus, einen verbesserten Aufschluss von Substratprotein, das dem Mikroorganismus während seiner Kultivierung zur Verfügung gestellt wird. Hierdurch stehen für die Synthese des Proteins mehr der nötigen Vorläufermoleküle, insbesondere Aminosäuren, zur Verfügung.

Dieser Sachverhalt wird ermittelt über die Transkriptionshäufigkeit der für das Protein codierenden Nukleinsäuresequenz und deren Translation in das gewünschte Protein, also über die Anzahl der pro Zeiteinheit erzeugten Proteine. Der Translationsschritt wird in die Bestimmung der Expression mit einbezogen. Als Vergleich dient ein Verfahren, welches sich von dem erfindungsgemäßen Verfahren durch die Abwesenheit der Hilfsprotease unterscheidet. Ein solcher Vergleich erfolgt erfindungsgemäß in Mikroorganismen gleichen Typs und unter gleichen Bedingungen, um die Vergleichbarkeit der Messungen zu gewährleisten.

Erfindungsgemäß ist das Verfahren dadurch gekennzeichnet, dass das Protein eine Protease, Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder eine Lipase ist. Ganz besonders bevorzugt handelt es sich bei dem Protein um eine Protease. Die zu produzierende Protease (= Zielprotease) ist dann gleichzeitig auch an der Hydrolyse des Protein-Substrats beteiligt und kann vorteilhafterweise einen nochmals verbesserten Aufschluss von Substratprotein bewirken.

Beispielsweise können mit einem erfindungsgemäßen Verfahren die nachstehend genannten Enzyme vorteilhaft hergestellt werden.

Unter den Proteasen sind Subtilisine bevorzugt. Beispiele hierfür sind die Subtilisine BPN' und Carlsberg, die Protease PB92, die Subtilisine 147 und 309, die Alkalische Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase® von der Firma Novozymes *A*/*S,* Bagsvrerd, Dänemark, erhältlich. Die Subtilisine 147 und 309 werden unter den Handelsnamen Esperase®, beziehungsweise Savinase® von der Firma Novozymes vertrieben.

Von der Protease aus *Bacillus lentus* DSM 5483 leiten sich die unter der Bezeichnung BLAP® geführten Protease-Varianten ab. Weitere bevorzugte Proteasen sind ferner beispielsweise die unter der Bezeichnung PUR geführten Enzyme. Weitere Proteasen sind ferner die unter den Handelsnamen Durazym®, Relase®, Everlase®, Nafizym®, Natalase®, Kannase® und Ovozyme® von der Firma Novozymes, die unter den Handelsnamen, Purafect®, Purafect® OxP, Purafect® Prime, Excellase® und Properase® von der Firma Genencor, das unter dem Handelsnamen Protosol® von der Firma Advanced Biochemicals Ltd., Thane, Indien, das unter dem Handelsnamen Wuxi® von der Firma Wuxi Snyder Bioproducts Ltd., China, die unter den Handelsnamen Proleather® und Protease P® von der Firma Amano Pharmaceuticals Ltd., Nagoya, Japan, und das unter der Bezeichnung Proteinase K-16 von der Firma Kao Corp., Tokyo, Japan, erhältlichen Enzyme. Bevorzugt sind ferner auch die Proteasen aus *Bacillus gibsonii* und *Bacillus pumilus,* die offenbart sind in den internationalen Patentanmeldungen WO 2008/086916 und WO 2007/131656.

Beispiele für Amylasen sind die α-Amylasen aus *Bacillus licheniformis,* aus *Bacillus amyloliquefaciens* oder aus *Bacillus stearothermophilus* sowie insbesondere auch deren für den Einsatz in Wasch- oder Reinigungsmitteln verbesserte Weiterentwicklungen. Das Enzym aus *Bacillus licheniformis* ist von dem Unternehmen Novozymes unter dem Namen Termamyl® und von dem Unternehmen Danisco/Genencor unter dem Namen Purastar®ST erhältlich. Weiterentwicklungsprodukte dieser α-Amylase sind von dem Unternehmen Novozymes unter den Handelsnamen Duramyl® und Termamyl®ultra, von dem Unternehmen Danisco/Genencor unter dem Namen Purastar®OxAm und von dem Unternehmen Daiwa Seiko Inc., Tokyo, Japan, als Keistase® erhältlich. Die α-Amylase von *Bacillus amyloliquefaciens* wird von dem Unternehmen Novozymes unter dem Namen BAN® vertrieben, und abgeleitete Varianten von der α-Amylase aus *Bacillus stearothermophilus* unter den Namen BSG® und Novamyl®, ebenfalls von dem Unternehmen Novozymes. Des Weiteren sind die α-Amylase aus *Bacillus sp.* A 7-7 (DSM 12368) und die Cyclodextrin-Glucanotransferase (CGTase) aus *Bacillus agaradherens* (DSM 9948) zu nennen. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus *Aspergillus nigerund A. oryzae* geeignet. Weitere vorteilhafte Handelsprodukte sind beispielsweise die Amylase Powerase® von dem Unternehmen Danisco/Genencor und die Amylasen Amylase-LT®, Stainzyme® und Stainzyme plus®, letztere von dem Unternehmen Novozymes. Auch durch Punktmutationen erhältliche Varianten dieser Enzyme können erfindungsgemäß hergestellt werden. Weitere bevorzugte Amylasen sind offenbart in den internationalen Offenlegungsschriften WO 00/60060, WO 03/002711, WO 03/054177 und WO 07/079938, auf deren Offenbarung daher ausdrücklich verwiesen wird bzw. deren diesbezüglicher Offenbarungsgehalt daher ausdrücklich in die vorliegende Patentanmeldung mit einbezogen wird. Erfindungsgemäß herzustellende Amylasen sind ferner vorzugsweise α-Amylasen.

Beispiele für Lipasen oder Cutinasen sind die ursprünglich aus *Humicola lanuginosa* (*Thermomyces lanuginosus*) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase®, Lipolase®Ultra, LipoPrime®, Lipozyme® und Lipex® vertrieben. Des Weiteren sind beispielsweise die Cutinasen herstellbar, die ursprünglich aus *Fusarium solani pisi* und *Humicola insolens* isoliert worden sind. Von der Firma Danisco/Genencor sind beispielsweise die Lipasen beziehungsweise Cutinasen herstellbar, deren Ausgangsenzyme ursprünglich aus *Pseudomonas mendocina* und *Fusarium solanii* isoliert worden sind. Als weitere wichtige Handelsprodukte sind die ursprünglich von der Firma Gist-Brocades (inzwischen Danisco/Genencor) vertriebenen Präparationen M1 Lipase® und Lipomax® und die von der Firma Meito Sangyo KK, Japan, unter den Namen Lipase MY-30®, Lipase OF® und Lipase PL® vertriebenen Enzyme zu erwähnen, ferner das Produkt Lumafast® von der Firma Danisco/Genencor.

Beispiele für Cellulasen (Endoglucanasen, EG) umfassen Sequenzen der pilzlichen, Endoglucanase(EG)-reichen Cellulase-Präparation beziehungsweise deren Weiterentwicklungen, die von dem Unternehmen Novozymes unter dem Handelsnamen Celluzyme® angeboten wird. Die ebenfalls von dem Unternehmen Novozymes erhältlichen Produkte Endolase® und Carezyme® basieren auf der 50 kD-EG, beziehungsweise der 43 kD-EG aus Humicola insolens DSM 1800. Weitere herstellbare Handelsprodukte dieses Unternehmens sind Cellusoft®, Renozyme® und Celluclean®. Weiterhin herstellbar sind beispielsweise Cellulasen, die von dem Unternehmen AB Enzymes, Finnland, unter den Handelsnamen Ecostone® und Biotouch® erhältlich sind, und die zumindest zum Teil auf der 20 kD-EG aus Melanocarpus basieren. Weitere Cellulasen von dem Unternehmen AB Enzymes sind Econase® und Ecopulp®. Weitere geeignete Cellulasen sind aus Bacillus sp. CBS 670.93 und CBS 669.93, wobei die aus Bacillus sp. CBS 670.93 von dem Unternehmen Danisco/Genencor unter dem Handelsnamen Puradax® erhältlich ist. Weitere herstellbare Handelsprodukte des Unternehmens Danisco/Genencor sind "Genencor detergent cellulase L" und IndiAge®Neutra.

Auch durch Punktmutationen erhältliche Varianten dieser Enzyme können erfindungsgemäß hergestellt werden. Besonders bevorzugte Cellulasen sind *Thielavia terrestris* Cellulasevarianten*,* die in der internationalen Offenlegungsschrift WO 98/12307 offenbart sind, Cellulasen aus *Melanocarpus,* insbesondere *Melanocarpus albomyces,* die in der internationalen Offenlegungsschrift WO 97/14804 offenbart sind, Cellulasen vom EGIII-Typ aus *Trichoderma reesei,* die in der europäischen Patentanmeldung EP 1305432 offenbart sind bzw. hieraus erhältliche Varianten, insbesondere diejenigen, die offenbart sind in den europäischen Patentanmeldungen EP 1240525 und EP 1305432, sowie Cellulasen, die offenbart sind in den internationalen Offenlegungsschriften WO 1992/006165, WO 96/29397 und WO 02/099091. Auf deren jeweilige Offenbarung wird daher ausdrücklich verwiesen bzw. deren diesbezüglicher Offenbarungsgehalt wird daher ausdrücklich in die vorliegende Patentanmeldung mit einbezogen.

Ferner können weitere Enzyme hergestellt werden, die unter dem Begriff Hemicellulasen zusammengefasst werden. Hierzu gehören beispielsweise Mannanasen, Xanthanlyasen, Xanthanasen, Pektinlyasen (=Pektinasen), Pektinesterasen, Pektatlyasen, Xyloglucanasen, Xylanasen, Pullulanasen und ß-Glucanasen. Diesbezüglich geeignete Enzyme sind beispielsweise unter den Namen Gamanase®, Pektinex AR® und Pectaway® von der Firma Novozymes, unter dem Namen Rohapec® 81 L von der Firma AB Enzymes und unter dem Namen Pyrolase® von der Firma Diversa Corp., San Diego, CA, USA erhältlich. Die aus Bacillus subtilis gewonnene ß- Glucanase ist unter dem Namen Cereflo® von der Firma Novozymes erhältlich. Erfindungsgemäß besonders bevorzugte Hemicellulasen sind Mannanasen, welche beispielsweise unter den Handelsnamen Mannaway® von dem Unternehmen Novozymes oder Purabrite® von dem Unternehmen Genencor vertrieben werden.

Ferner können auch Oxidoreduktasen, beispielsweise Oxidasen, Oxygenasen, Katalasen, Peroxidasen, wie Halo-, Chloro-, Bromo-, Lignin-, Glucose- oder Manganperoxidasen, Dioxygenasen oder Laccasen (Phenoloxidasen, Polyphenoloxidasen) hergestellt werden. Als geeignete Handelsprodukte sind Denilite® 1 und 2 der Firma Novozymes zu nennen. Weitere Enzyme sind in den internationalen Patentanmeldungen WO 98/45398, WO 2005/056782, WO 2004/058961 sowie WO 2005/124012 offenbart.

In einer weiteren Ausführungsform der Erfindung ist das Verfahren dadurch gekennzeichnet, dass der Mikroorganismus ein Bakterium ist.

Bakterien sind bevorzugte Mikroorganismen in erfindungsgemäßen Verfahren. Bakterien zeichnen sich durch kurze Generationszeiten und geringe Ansprüche an die Kultivierungsbedingungen aus. Dadurch können kostengünstige Kultivierungsverfahren bzw. Herstellungsverfahren etabliert werden. Zudem verfügt der Fachmann bei Bakterien in der Fermentationstechnik über einen reichhaltigen Erfahrungsschatz. Es können sowohl gramnegative als auch grampositive Bakterien geeignet sein.

Bei gramnegativen Bakterien wie beispielsweise *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Mikroorganismen wie *Actinomyceten* oder andere Vertreter der *Actinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden.

Ein erfindungsgemäß bevorzugtes Bakterium ist ausgewählt aus der Gruppe der Gattungen von *Escherichia, Klebsiella, Burkholderia, Bacillus, Staphylococcus, Corynebakterium, Arthrobacter, Streptomyces, Stenotrophomonas, Pseudomonas* und *Vibrio.* Weiter bevorzugt ist es eines, das ausgewählt ist aus der Gruppe von *Escherichia coli, Klebsiella planticola, Burkholderia glumae, Bacillus licheniformis, Bacillus lentus, Bacillus amyloliquefaciens, Bacillus subtilis, Bacillus alcalophilus, Bacillus globigii, Bacillus gibsonii*, *Bacillus pumilus, Staphylococcus carnosus, Corynebacterium glutamicum, Arthrobacter oxidans, Streptomyces lividans, Streptomyces coelicolor, Stenotrophomonas maltophilia* und *Vibrio, metschnikovii.* Ganz besonders bevorzugt ist *Bacillus licheniformis.*

Der Mikroorganismus kann aber auch eine eukaryontische Zelle sein, die dadurch gekennzeichnet ist, dass sie einen Zellkern besitzt. Im Gegensatz zu prokaryontischen Zellen sind eukaryontische Zellen in der Lage, das gebildete Protein posttranslational zu modifizieren. Beispiele dafür sind Pilze wie *Actinomyceten* oder Hefen wie *Saccharomyces* oder *Kluyveromyces.* Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen oder Lipasen, kann vorteilhaft sein. Ferner können sich beispielsweise thermophile pilzliche Expressionssysteme besonders zur Expression temperaturbeständiger Proteine oder Varianten eignen.

Erfindungsgemäß einzusetzende Mikroorganismen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Mikroorganismen handeln, die mehrere Zielproteine transgen exprimieren. Bevorzugt sezernieren sie das Protein (Zielprotein) in das den Mikroorganismus umgebende Medium.

Die in erfindungsgemäßen Verfahren zum Einsatz kommenden Mikroorganismen können in üblicher Weise kultiviert und fermentiert werden, beispielsweise in diskontinuierlichen oder kontinuierlichen Systemen. Im ersten Fall wird ein geeignetes Nährmedium mit den Mikroorganismen beimpft und das Protein nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Verfahren sind vorzugsweise Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Proteins. Alle Fermentationsverfahren, die auf einem erfindungsgemäßen Verfahren zur Herstellung eines Proteins beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse bzw. Trockenmasse erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Das hergestellte Protein kann aus dem Fermentationsmedium geerntet werden. Ein solches Fermentationsverfahren ist gegenüber einer Isolation des Proteins aus dem Mikroorganismus, d.h. einer Produktaufbereitung aus der Zellmasse (Trockenmasse) bevorzugt. Dies kann beispielsweise erreicht werden durch die Zurverfügungstellung von geeigneten Mikroorganismen oder von einem oder mehreren geeigneten Sekretionsmarkern bzw. -mechanismen und/oder Transportsystemen, damit die Mikroorganismen das Protein in das Fermentationsmedium sezernieren. Ohne Sekretion kann alternativ die Isolation des Proteins aus der Wirtszelle, d.h. eine Aufreinigung desselben aus der Zellmasse, erfolgen, beispielsweise durch Fällung mit Ammoniumsulfat oder Ethanol, oder durch chromatographische Reinigung.

Ein weiterer Gegenstand der Erfindung ist ein Mikroorganismus, der durch ein Verfahren umfassend die Verfahrensschritte
(a) Einbringen eines ersten Expressionskonstruktes in den Mikroorganismus, welches für ein Protein codiert, wobei das Protein eine Protease, Amylase, Cellulase, Hemicellulase, Mannanase, Tannase, Xylanase, Xanthanase, Xyloglucanase, [beta]-Glucosidase, Pektinase, Carrageenase, Perhydrolase, Oxidase, Oxidoreduktase oder eine Lipase ist;
(b) Einbringen eines zweiten Expressionskonstruktes in den Mikroorganismus, welches für eine Hilfsprotease codiert, die sich von dem Protein unterscheidet und die eine Aminosäuresequenz umfasst, die zu der in SEQ ID NO. 1 (= Blap S) angegebenen Aminosäuresequenz zu mindestens 65% identisch ist, wobei die Hilfsprotease eine proteolytische Aktivität aufweist und mindestens eine chromosomal codierte Protease in dem Mikroorganismus ersetzt, erhältlich ist.

Hierbei handelt es sich demnach um alle Mikroorganismen, die Gegenstand eines erfindungsgemäßen Verfahrens sein können. Alle Sachverhalte, Gegenstände und Ausführungsformen, die für erfindungsgemäße Verfahren beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die erfindungsgemäßen Mikroorganismen gilt.

Besonders bevorzugte Ausführungsformen erfindungsgemäßer Mikroorganismen sind dadurch gekennzeichnet, dass
(a) das Protein nicht natürlicherweise in dem Mikroorganismus vorhanden ist und/oder
(b) die Hilfsprotease nicht natürlicherweise in dem Mikroorganismus vorhanden ist und/oder
(c) die Hilfsprotease in dem Mikroorganismus zusätzlich zu den chromosomal codierten Proteasen exprimiert wird, und/oder
(d) die Expression der Hilfsprotease die Expression des Proteins verstärkt, und/oder
(e) der Mikroorganismus ein Bakterium ist, bevorzugt eines, das ausgewählt ist aus der Gruppe der Gattungen von *Escherichia, Klebsiella, Burkholderia, Bacillus, Staphylococcus, Corynebakterium, Arthrobacter, Streptomyces, Stenotrophomonas, Pseudomonas* und *Vibrio,* weiter bevorzugt eines, das ausgewählt ist aus der Gruppe von *Escherichia coli, Klebsiella planticola, Burkholderia glumae, Bacillus licheniformis, Bacillus lentus, Bacillus amyloliquefaciens, Bacillus subtilis, Bacillus alcalophilus, Bacillus globigii*, *Bacillus gibsonii, Bacillus pumilus, Staphylococcus carnosus, Corynebacterium glutamicum, Arthrobacter oxidans, Streptomyces lividans, Streptomyces coelicolor, Stenotrophomonas maltophilia* und *Vibrio, metschnikovii.* Ganz besonders bevorzugt ist *Bacillus licheniformis.*

Erfindungsgemäße Mikroorganismen werden vorteilhaft in erfindungsgemäßen Verfahren verwendet, um ein Protein herzustellen, wobei das Protein eine Protease, Amylase, Cellulase, Hemicellulase, Mannanase, Tannase, Xylanase, Xanthanase, Xyloglucanase, [beta]-Glucosidase, Pektinase, Carrageenase, Perhydrolase, Oxidase, Oxidoreduktase oder eine Lipase ist.

Alle Sachverhalte, Gegenstände und Ausführungsformen, die für erfindungsgemäße Verfahren bzw. Mikroorganismen beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die erfindungsgemäßen Verwendungen gilt.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme, Baukästen (Kits) und Geräte wurden nach den Angaben der jeweiligen Hersteller eingesetzt.

### Beispiel 1:

Substitution des Gens, welches für eine Protease gemäß SEQ ID NO. 2 codiert (aprE), durch ein Gen, das für eine Hilfsprotease gemäß SEQ ID NO. 1 codiert, in *Bacillus licheniformis.*

Folgende Oligonukleotide wurden als Primer (angegeben in 5'→ 3' Orientierung) verwendet:

| | |
|---|---|
| aprE1 (SEQ ID NO. 3) | AAACTGCAGCGTACCGGCTCCTTGAAAGG |
| aprE2 (SEQ ID NO. 4) | |
| aprE3 (SEQ ID NO. 5) | |
| aprE4 (SEQ ID NO. 6) | AAATCTAGATAGACCTCGAAGAGGAGAAGC |
| tet5 (SEQ ID NO. 7) | GCGGATCCAAACGGGCCATATTGTTGTATAAG |
| tet6 (SEQ ID NO. 8) | CCAAGCTTCTCTCGTATCTTTTATTCAGCAATCGC |
| aprE7 (SEQ ID NO.9) | |
| aprE8 (SEQ ID NO. 10) | |
| ziel9 (SEQ ID NO. 11) | |
| ziel10 (SEQ ID NO. 12) | |

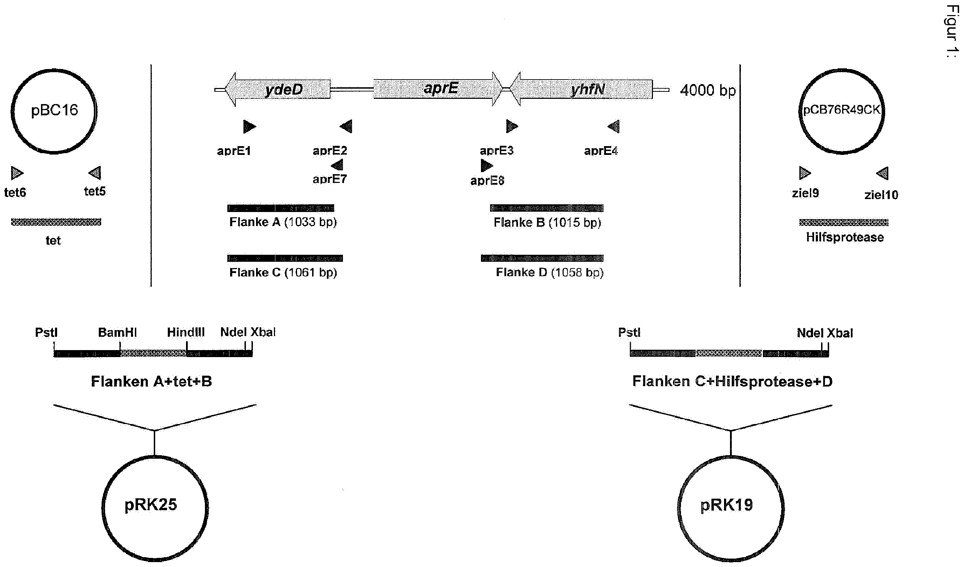

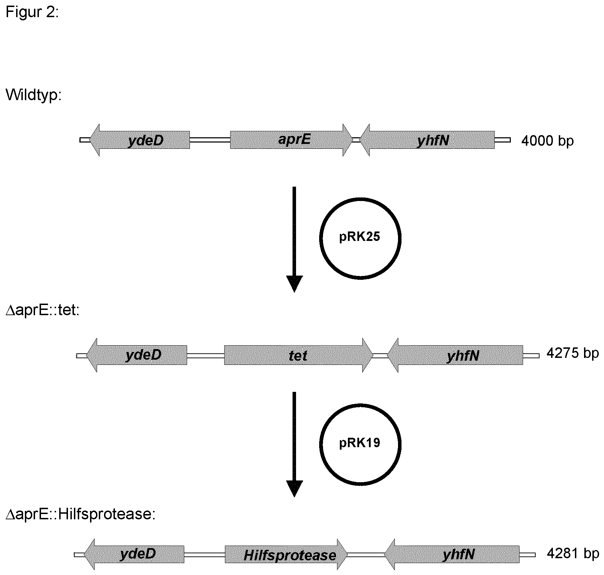

Der Austausch erfolgte mit einem Zwischenschritt, in dem zunächst das aprE-Gen gegen das Tetracyclin-Resistenzgen tet ausgetauscht wurde. Zur Substitution des aprE-Gens gegen das tet- Gen wurden mittels der Primer aprE1 und aprE2 sowie der Primer aprE3 und aprE4 Sequenzabschnitte von 1033 bp bzw. 1015 bp upstream und downstream vom aprE-Gen amplifiziert (Flanken A und B). Aufgrund entsprechender überhänge der Primer aprE2 und aprE3 wurden durch Fusions-Polymerase-Kettenreaktion (PCR) mittels der äußeren Primer aprE1 und aprE4 die beiden Flanken fusioniert. Das 2018 bp Fusions-PCR-Produkt wurde in die Schnittstellen Pstl und Xbal des Vektors pE194 (Horinouchi & Weisblum, J.Bacteriol. (150), S. 804ff (1982)) kloniert. Das tet-Gen wurde mittels der Primer tet5 und tet6 aus dem Plasmid pBC16 (Bernhard et al., J.Bacteriol. (133), S. 897ff, (1978)) amplifiziert und in die zentralen Schnittstellen BamHI und HindIII der fusionierten aprE-Flanken kloniert (vgl. Figur 1). Das erhaltene Plasmid pRK25 wurde in Bacillus licheniformis transformiert. Die Selektion erfolgte sowohl mit 5 µg/ml Erythromycin als auch mit 15 µg/ml Tetracyclin bei 30°C. Nach Kultivierung bei 42°C wurden Klone mit integriertem Vektor durch Wachstum auf 0,3 µg/ml Erythromycin identifiziert. Nach erneuter Kultivierung bei 42°C jedoch ohne Einsatz von Erythromycin wurden Klone erhalten, die auf Tetracyclin-Resistenz und mittels PCR auf den erfolgreichen Austausch des aprE-Gens gegen das tet-Gen gescreent wurden (*Bacillus licheniformis* ΔaprE::tet*,* vgl. Figur 2).

Zur Substitution des tet-Gens gegen das Gen, das für eine Hilfsprotease gemäß SEQ ID NO. 1 codiert, wurden mittels der Primer aprE1 und aprE7 sowie der Primer aprE8 und aprE4 Sequenzabschnitte von 1061 bp bzw. 1058 bp upstream und downstream vom aprE-Gen amplifiziert (Flanken C und D). Das Gen, das für eine Hilfsprotease gemäß SEQ ID NO. 1 codiert, wurde mittels der Primer ziel9 und ziel10 aus dem Plasmid pCB76R49CK amplifiziert. Aufgrund entsprechender überhänge der Primer aprE7 und ziel9 bzw. aprE8 und ziel10 wurden durch Fusions-PCR mittels der äußeren Primer aprE1 und aprE4 die beiden Flanken mit dem Hilfsprotease-PCR-Produkt fusioniert. Das 3704 bp Fusions-PCR-Produkt wurde in die Schnittstellen Pstl und Ndel des Vektors pRK25 kloniert (vgl. Figur 1). Das erhaltene Plasmid pRK19 wurde in *Bacillus licheniformis* ΔaprE:tet (s.o.) transformiert. Die Selektion erfolgte mit 5 µg/ml Erythromycin bei 30°C. Nach Kultivierung bei 42°C wurden Klone mit integriertem Vektor durch Wachstum auf 0,3 µg/ml Erythromycin identifiziert. Nach erneuter Kultivierung bei 42°C jedoch ohne Einsatz von Antibiotikum wurden Klone erhalten, die mittels PCR auf den erfolgreichen Austausch des tet-Gens gegen das Gen, das für eine Hilfsprotease gemäß SEQ ID NO. 1 codiert, gescreent wurden (*Bacillus licheniformis* ΔaprE::Hilfsprotease, vgl. Figur 2).

### Beispiel 2:

Fermentative Produktion einer Protease (Zielprotein) mit Hilfe des Produktionsstamms *Bacillus licheniformis* ΔaprE::Hilfsprotease.

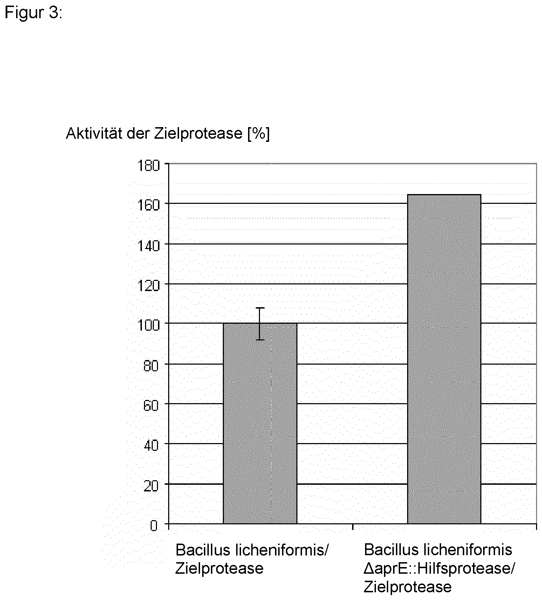

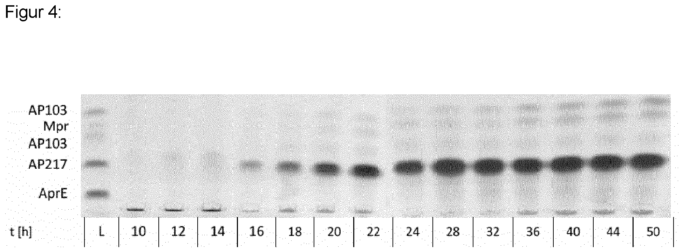

Der *Bacillus licheniformis* Ausgangsstamm sowie die Substitutionsmutante Δ aprE::Hilfsprotease wurden mit einem Produktionsplasmid für eine Protease (Zielprotein) transformiert. Die resultierenden Produktionsstämme wurden in einem Standardfermentationsverfahren eingesetzt und die erhaltenen Proteaseaktivitäten bestimmt. Verglichen mit dem Ausgangsstamm stieg die Ausbeute im 2 Liter-Laborfermenter um 65% (vgl. Figur 3). Die gemessenen Proteaseaktivitäten beruhen im Wesentlichen auf der Aktivität der Zielprotease, da die Expression der Zielprotease deutlich gesteigert wurde. Dies wurde durch eine Sekretionsanalyse des Fermentationsüberstandes der Kultur von *Bacillus licheniformis* ΔaprE::Hilfs-protease/Zielprotease mittels nativer Polyacrylamid-Gelelektrophorese verifiziert (vgl. Figur 4; t beschreibt die Kultivierungszeit, AP103 ist die Hilfsprotease (zwei Banden), AP217 ist die Zielprotease). Im Vergleich zur Zielprotease liegt nur eine sehr geringe Menge an Hilfsprotease im Kulturüberstand vor.

### Beschreibung der Figuren

Figur 1: Schematische Darstellung der Konstruktion der Vektoren pRK25 und pRK19 für den chromosomalen Austausch des Gens, welches für eine Protease gemäß SEQ ID NO. 2 codiert (aprE), gegen das Tetracyclin-Resistenzgen tet bzw. das Gen, das für eine Hilfsprotease gemäß SEQ ID NO. 1 codiert. Pfeile kennzeichnen die Leserahmen der Gene ydeD, aprE und yhfN, kennzeichnen Primer, Balken kennzeichnen PCR-Produkte.
Figur 2: Genort des aprE-Gens in *Bacillus licheniformis.* Dargestellt ist die Wildtyp-Situation (oben), der pRK19-vermittelte Austausch des aprE-Gens gegen das Tetracyclin-Resistenzgen tet (Mitte) und der pRK25-vermittelte Austausch des Tetracyclin-Resistenzgens tet gegen das Gen, das für eine Hilfsprotease gemäß SEQ ID NO. 1 codiert (unten).
Figur 3: Relative Ausbeuten der Produktionsstämme *Bacillus licheniformisl* Zielprotease und *Bacillus licheniformis* ΔaprE::Hilfsprotease/Zielprotease*.* Die Stämme wurden in einem Standardfermentationsverfahren zur Produktion der Zielprotease eingesetzt.
Figur 4: Sekretionsanalyse des Fermentationsüberstandes in einem erfindungsgemäßen Verfahren bzw. bei einem erfindungsgemäßen Mikroorganismus (*Bacillus licheniformis* ΔaprE::Hilfsprotease/Zielprotease*,* vgl. auch Figur 3) mittels nativer Polyacrylamid-Gelelektrophorese. Die gesteigerte Proteaseaktivität beruht im Wesentlichen auf der Aktivität der Zielprotease, da deren Expression und Sekretion deutlich gesteigert wurde (t: Kultivierungszeit, AP103: Hilfsprotease (zwei Banden), AP217: Zielprotease, Mpr: weiterer Größenmarker).

## Patentansprüche

1. Verfahren zur Herstellung eines Proteins durch einen Mikroorganismus umfassend die Verfahrensschritte
(a) Einbringen eines ersten Expressionskonstruktes in einen Mikroorganismus, welches für das Protein codiert;
(b) Einbringen eines zweiten Expressionskonstruktes in den Mikroorganismus, welches für eine Hilfsprotease codiert, die sich von dem Protein unterscheidet und die eine Aminosäuresequenz umfasst, die zu der in SEQ ID NO. 1 angegebenen Aminosäuresequenz zu mindestens 65% identisch ist, wobei die Hilfsprotease eine proteolytische Aktivität aufweist und mindestens eine chromosomal codierte Protease in dem Mikroorganismus ersetzt;
(c) Exprimieren des Proteins und der Hilfsprotease in dem Mikroorganismus, wobei das Protein eine Protease, Amylase, Cellulase, Hemicellulase, Mannanase, Tannase, Xylanase, Xanthanase, Xyloglucanase, [beta]-Glucosidase, Pektinase, Carrageenase, Perhydrolase, Oxidase, Oxidoreduktase oder eine Lipase ist.

2. Verfahren nach Anspruch 1, **dadurch gekennzeichnet, dass** das Protein nicht natürlicherweise in dem Mikroorganismus vorhanden ist und/oder die Hilfsprotease nicht natürlicherweise in dem Mikroorganismus vorhanden ist.

3. Verfahren nach Anspruch 1, **dadurch gekennzeichnet, dass** die Hilfsprotease mindestens eine chromosomal codierte Protease in dem Mikroorganismus ersetzt, die eine Aminosäuresequenz umfasst, die zu der in SEQ ID NO. 2 angegebenen Aminosäuresequenz zu mindestens 80% identisch ist.

4. Verfahren nach einem der Ansprüche 1 bis 3, **dadurch gekennzeichnet, dass** die Expression des Proteins durch die Expression der Hilfsprotease verstärkt wird.

5. Verfahren nach einem der Ansprüche 1 bis 4, **dadurch gekennzeichnet, dass** der Mikroorganismus ein Bakterium ist, bevorzugt eines, das ausgewählt ist aus der Gruppe der Gattungen von Escherichia, Klebsiella, Burkholderia, Bacillus, Staphylococcus, Corynebakterium, Arthrobacter, Streptomyces, Stenotrophomonas, Pseudomonas und Vibrio, weiter bevorzugt eines, das ausgewählt ist aus der Gruppe von Escherichia coli, Klebsiella planticola, Burkholderia glumae, Bacillus licheniformis, Bacillus lentus, Bacillus amyloliquefaciens, Bacillus subtilis, Bacillus alcalophilus, Bacillus globigii, Bacillus gibsonii, Bacillus pumilus, Staphylococcus carnosus, Corynebacterium glutamicum, Arthrobacter oxidans, Streptomyces lividans, Streptomyces coelicolor, Stenotrophomonas maltophilia und Vibrio metschnikovii.

6. Mikroorganismus, erhältlich durch ein Verfahren umfassend die Verfahrensschritte
(a) Einbringen eines ersten Expressionskonstruktes in den Mikroorganismus, welches für ein Protein codiert, wobei das Protein eine Protease, Amylase, Cellulase, Hemicellulase, Mannanase, Tannase, Xylanase, Xanthanase, Xyloglucanase, [beta]-Glucosidase, Pektinase, Carrageenase, Perhydrolase, Oxidase, Oxidoreduktase oder eine Lipase ist;
(b) Einbringen eines zweiten Expressionskonstruktes in den Mikroorganismus, welches für eine Hilfsprotease codiert, die sich von dem Protein unterscheidet und die eine Aminosäuresequenz umfasst, die zu der in SEQ ID NO. 1 angegebenen Aminosäuresequenz zu mindestens 65% identisch ist, wobei die Hilfsprotease eine proteolytische Aktivität aufweist und mindestens eine chromosomal codierte Protease in dem Mikroorganismus ersetzt.

7. Mikroorganismus nach Anspruch 6, **dadurch gekennzeichnet, dass**
(a) das Protein nicht natürlicherweise in dem Mikroorganismus vorhanden ist und/oder
(b) die Hilfsprotease nicht natürlicherweise in dem Mikroorganismus vorhanden ist und/oder
(c) die Expression der Hilfsprotease die Expression des Proteins verstärkt, und/oder
(d) der Mikroorganismus ein Bakterium ist, bevorzugt eines, das ausgewählt ist aus der Gruppe der Gattungen von Escherichia, Klebsiella, Burkholderia, Bacillus, Staphylococcus, Corynebakterium, Arthrobacter, Streptomyces, Stenotrophomonas, Pseudomonas und Vibrio, weiter bevorzugt eines, das ausgewählt ist aus der Gruppe von Escherichia coli, Klebsiella planticola, Burkholderia glumae, Bacillus licheniformis, Bacillus lentus, Bacillus amyloliquefaciens, Bacillus subtilis, Bacillus alcalophilus, Bacillus globigii, Bacillus gibsonii, Bacillus pumilus, Staphylococcus carnosus, Corynebacterium glutamicum, Arthrobacter oxidans, Streptomyces lividans, Streptomyces coelicolor, Stenotrophomonas maltophilia und Vibrio metschnikovii.

8. Verwendung eines Mikroorganismus nach einem der Ansprüche 6 oder 7 zur Herstellung einer Protease, Amylase, Cellulase, Hemicellulase, Mannanase, Tannase, Xylanase, Xanthanase, Xyloglucanase, [beta]-Glucosidase, Pektinase, Carrageenase, Perhydrolase, Oxidase, Oxidoreduktase oder einer Lipase.

## Claims

1. A method for preparing a protein by means of a microorganism comprising the method steps of
(a) introducing into a microorganism a first expression construct which encodes the protein;
(b) introducing into the microorganism a second expression construct which encodes an auxiliary protease which differs from the protein and which comprises an amino acid sequence which is at least 65% identical to the amino acid sequence indicated in SEQ ID NO. 1, wherein the auxiliary protease has proteolytic activity and replaces at least one chromosome-encoded protease in the microorganism;
(c) expressing the protein and the auxiliary protease in the microorganism, where the protein is a protease, amylase, cellulase, hemicellulase, mannanase, tannase, xylanase, xanthanase, xyloglucanase, [beta]-glucosidase, pectinase, carrageenase, perhydrolase, oxidase, oxidoreductase or a lipase.

2. The method according to claim 1, wherein the protein is not naturally present in the microorganism and/or the auxiliary protease is not naturally present in the microorganism.

3. The method according to claim 1, wherein the auxiliary protease replaces in the microorganism at least one chromosome-encoded protease which comprises an amino acid sequence which is at least 80% identical to the amino acid sequence indicated in SEQ ID NO. 2.

4. The method according to any of claims 1 to 3, wherein the expression of the protein is enhanced by the expression of the auxiliary protease.

5. The method according to any of claims 1 to 4, wherein the microorganism is a bacterium, preferably one selected from the group of the genera of Escherichia, Klebsiella, Burkholderia, Bacillus, Staphylococcus, Corynebakterium, Arthrobacter, Streptomyces, Stenotrophomonas, Pseudomonas and Vibrio, more preferably one selected from the group of Escherichia coli, Klebsiella planticola, Burkholderia glumae, Bacillus licheniformis, Bacillus lentus, Bacillus amyloliquefaciens, Bacillus subtilis, Bacillus alcalophilus, Bacillus globigii, Bacillus gibsonii, Bacillus pumilus, Staphylococcus carnosus, Corynebacterium glutamicum, Arthrobacter oxidans, Streptomyces lividans, Streptomyces coelicolor, Stenotrophomonas maltophilia and Vibrio metschnikovii.

6. A microorganism obtainable by a method comprising the method steps of
(a) introducing into the microorganism a first expression construct which encodes a protein, where the protein is a protease, amylase, cellulase, hemicellulase, mannanase, tannase, xylanase, xanthanase, xyloglucanase, [beta]-glucosidase, pectinase, carrageenase, perhydrolase, oxidase, oxidoreductase or a lipase;
(b) introducing into the microorganism a second expression construct which encodes an auxiliary protease which differs from the protein and which comprises an amino acid sequence which is at least 65% identical to the amino acid sequence indicated in SEQ ID NO.1, wherein the auxiliary protease has proteolytic activity and replaces at least one chromosome-encoded protease in the microorganism.

7. The microorganism according to claim 6, wherein
(a) the protein is not naturally present in the microorganism and/or
(b) the auxiliary protease is not naturally present in the microorganism and/or
(c) the expression of the auxiliary protease enhances the expression of the protein, and/or
(d) the microorganism is a bacterium, preferably one selected from the group of the genera of Escherichia, Klebsiella, Burkholderia, Bacillus, Staphylococcus, Corynebakterium, Arthrobacter, Streptomyces, Stenotrophomonas, Pseudomonas and Vibrio, more preferably one selected from the group of Escherichia coli, Klebsiella planticola, Burkholderia glumae, Bacillus licheniformis, Bacillus lentus, Bacillus amyloliquefaciens, Bacillus subtilis, Bacillus alcalophilus, Bacillus globigii, Bacillus gibsonii, Bacillus pumilus, Staphylococcus carnosus, Corynebacterium glutamicum, Arthrobacter oxidans, Streptomyces lividans, Streptomyces coelicolor, Stenotrophomonas maltophilia and Vibrio metschnikovii.

8. Use of a microorganism according to either of claims 6 and 7 for preparing a protease, amylase, cellulase, hemicellulase, mannanase, tannase, xylanase, xanthanase, xyloglucanase, [beta]-glucosidase, pectinase, carrageenase, perhydrolase, oxidase, oxidoreductase or a lipase.

## Revendications

1. Procédé de fabrication d'une protéine par un microorganisme, comprenant les étapes de procédé suivantes :
(a) l'introduction d'une première construction d'expression dans un microorganisme, qui code pour la protéine ;
(b) l'introduction d'une deuxième construction d'expression dans le microorganisme, qui code pour une protéase auxiliaire différente de la protéine et qui comprend une séquence d'acides aminés qui est au moins 65 % identique à la séquence d'acides aminés indiquée dans SEQ ID NO. 1, la protéase auxiliaire présentant une activité protéolytique et remplaçant au moins une protéase codée chromosomiquement dans le microorganisme ;
(c) l'expression de la protéine et de la protéase auxiliaire dans le microorganisme, la protéine étant une protéase, une amylase, une cellulase, une hémicellulase, une mannanase, une tannase, une xylanase, une xanthanase, une xyloglucanase, une [bêta]-glucosidase, une pectinase, une carraghénase, une perhydrolase, une oxydase, une oxydoréductase ou une lipase.

2. Procédé selon la revendication 1, **caractérisé en ce que** la protéine n'est pas présente naturellement dans le microorganisme et/ou la protéase auxiliaire n'est pas présente naturellement dans le microorganisme.

3. Procédé selon la revendication 1, **caractérisé en ce que** la protéase auxiliaire remplace au moins une protéase codée chromosomiquement dans le microorganisme, qui comprend une séquence d'acides aminés qui est au moins 80 % identique à la séquence d'acides aminés indiquée dans SEQ ID NO. 2.

4. Procédé selon l'une quelconque des revendications 1 à 3, **caractérisé en ce que** l'expression de la protéine est renforcée par l'expression de la protéase auxiliaire.

5. Procédé selon l'une quelconque des revendications 1 à 4, **caractérisé en ce que** le microorganisme est une bactérie, de préférence une bactérie qui est choisie dans le groupe des genres Escherichia, Klebsiella, Burkholderia, Bacillus, Staphylococcus, Corynebacterium, Arthrobacter, Streptomyces, Stenotrophomonas, Pseudomonas et Vibrio, de manière davantage préférée une bactérie qui est choisie dans le groupe constitué par Escherichia coli, Klebsiella planticola, Burkholderia glumae, Bacillus licheniformis, Bacillus lentus, Bacillus amyloliquefaciens, Bacillus subtilis, Bacillus alcalophilus, Bacillus globigii, Bacillus gibsonii, Bacillus pumilus, Staphylococcus camosus, Corynebacterium glutamicum, Arthrobacter oxidans, Streptomyces lividans, Streptomyces coelicolor, Stenotrophomonas maltophilia et Vibrio metschnikovii.

6. Microorganisme, pouvant être obtenu par un procédé comprenant les étapes de procédé suivantes :
(a) l'introduction d'une première construction d'expression dans le microorganisme, qui code pour une protéine, la protéine étant une protéase, une amylase, une cellulase, une hémicellulase, une mannanase, une tannase, une xylanase, une xanthanase, une xyloglucanase, une [bêta]-glucosidase, une pectinase, une carraghénase, une perhydrolase, une oxydase, une oxydoréductase ou une lipase ;
(b) l'introduction d'une deuxième construction d'expression dans le microorganisme, qui code pour une protéase auxiliaire différente de la protéine et qui comprend une séquence d'acides aminés qui est au moins 65 % identique à la séquence d'acides aminés indiquée dans SEQ ID NO. 1, la protéase auxiliaire présentant une activité protéolytique et remplaçant au moins une protéase codée chromosomiquement dans le microorganisme.

7. Microorganisme selon la revendication 6, **caractérisé en ce que**
(a) la protéine n'est pas présente naturellement dans le microorganisme et/ou
(b) la protéase auxiliaire n'est pas présente naturellement dans le microorganisme et/ou
(c) l'expression de la protéase auxiliaire renforce l'expression de la protéine et/ou
(d) le microorganisme est une bactérie, de préférence une bactérie qui est choisie dans le groupe des genres Escherichia, Klebsiella, Burkholderia, Bacillus, Staphylococcus, Corynebacterium, Arthrobacter, Streptomyces, Stenotrophomonas, Pseudomonas et Vibrio, de manière davantage préférée une bactérie qui est choisie dans le groupe constitué par Escherichia coli, Klebsiella planticola, Burkholderia glumae, Bacillus licheniformis, Bacillus lentus, Bacillus amyloliquefaciens, Bacillus subtilis, Bacillus alcalophilus, Bacillus globigii, Bacillus gibsonii, Bacillus pumilus, Staphylococcus camosus, Corynebacterium glutamicum, Arthrobacter oxidans, Streptomyces lividans, Streptomyces coelicolor, Stenotrophomonas maltophilia et Vibrio metschnikovii.

8. Utilisation d'un microorganisme selon l'une quelconque des revendications 6 ou 7 pour la fabrication d'une protéase, d'une amylase, d'une cellulase, d'une hémicellulase, d'une mannanase, d'une tannase, d'une xylanase, d'une xanthanase, d'une xyloglucanase, d'une [bêta]-glucosidase, d'une pectinase, d'une carraghénase, d'une perhydrolase, d'une oxydase, d'une oxydoréductase ou d'une lipase.
